# EUROPEAN PATENT APPLICATION

(11) **EP 4 682 217 A1**
(43) Date of publication of application: **21.01.2026**
(21) Application number: 24770552.8
(22) Date of filing: 29.02.2024
(51) Int. Cl.: C09K 3/00, A41D 13/00, A41D 31/00, A41D 31/04, D01F 1/10

(54) **FIRED OBJECT**

(30) Priority: 15.03.2023 JP 2023041075
(71) Applicant: Komuro, Yoshiko, Tokyo 176-0011 (JP)
(72) Inventor: KOMURO, Toshio, Tokyo 176-0011 (JP)
(74) Representative: Müller-Boré & Partner Patentanwälte PartG mbB
(86) International application number: PCT/JP2024/007590
(87) International publication number: WO 2024/190441

(57) **Abstract**

Provided is a calcined product which generates photons. The calcined product of the present invention is a calcined product of a composition which comprises (i) alumina, (ii) at least one kind selected from the group consisting of silica and titanium oxide, (iii) at least one kind selected from the group consisting of platinum, a platinum compound, palladium, a palladium compound, iridium, an iridium compound, rhodium, and a rhodium compound, and (iv) silicon nitride, wherein the composition contains the silicon nitride (iv) in an amount of 5 to 10 parts by mass, in 100 parts by mass of the composition.

## Description

### Field of the Invention

The present invention relates to a calcined product.

### Background Art

It has been widely known that a composition containing platinum generates a far infrared ray. A far infrared ray is an electromagnetic wave having a radiation wavelength in the range of several to about 400 µm, and has excellent heating effect and drying effect. For this reason, a ceramic as a far infrared ray radiator can uniformly heat the inside of an object without excessively heating the surface, and thus has been used in the field of high-quality food processing. Further, fibers having a far infrared effect, which have a far infrared ray radiator incorporated into a fiber or applied to the surface of a fiber, are widely used in, for example, bedding articles, clothing, and underwear.

For example, Japanese Unexamined Patent Publication No. Sho 62-184088 has a description that a far infrared ray radiating powder comprising alumina, silica, and platinum is effective in improving the shelf life or taste of foods.

Japanese Unexamined Patent Publication No. Hei 3-190990 has a description that an infrared weak energy radiating powder comprising alumina, titanium, and platinum is effective in improving the shelf life of foods, and that a synthetic fiber containing the powder promotes blood circulation and has a warming effect, and thus is effective in treating a symptom of being sensitive to the cold or arthritis pain.

Further, Japanese Unexamined Patent Publication Nos. Hei 3-241025 and Hei 4-73226 have a description that woven fabric produced from a yarn obtained by mixing a far infrared ray radiating powder comprising alumina, silica, and platinum into nylon or polyester has extremely excellent heat insulating properties for living body.

Thereafter, there has been reported a composition comprising platinum, wherein the composition generates photons and negative ions as well as a far infrared ray.

For example, patent document 5 has a description that a composition comprising alumina, silica, titanium oxide, platinum, and silver has been found to generate a far infrared ray and photons, and that the composition has excellent antifungal and bactericidal properties and a fiber product having the composition mixed into a fiber has antistatic properties.

Patent document 6 has a description that a composition comprising alumina, silica, titanium oxide, platinum, and silver generates negative ions and exhibits antifungal properties, and that antithrombotic properties are obtained by using clothing or bedding articles using a fiber containing the composition.

Patent document 7 has a description that a calcined product of a composition, which comprises alumina, at least one kind selected from the group consisting of silica and titanium oxide, at least one kind selected from the group consisting of platinum, a platinum compound, palladium, a palladium compound, iridium, an iridium compound, rhodium, and a rhodium compound, celsian, and steatite, generates photons and ions and exhibits effects for promotion of health, such as a reduction of the lactate concentration of a blood, an improvement of immunity, or a reduction of lipoperoxide.

However, the above-mentioned compositions or calcined products are desired to be improved in the amount of photons generated therefrom.

### PRIOR ART REFERENCES

### Patent Documents

Patent document 1: Japanese Unexamined Patent Publication No. Sho 62-184088
Patent document 2: Japanese Unexamined Patent Publication No. Hei 3-190990
Patent document 3: Japanese Unexamined Patent Publication No. Hei 3-241025
Patent document 4: Japanese Unexamined Patent Publication No. Hei 4-73226
Patent document 5: Japanese Patent No. 4195562
Patent document 6: Japanese Patent No. 4712378
Patent document 7: Japanese Patent No. 6606213

### Summary of the Invention

### Problems to be Solved by the Invention

An object of the present invention is to provide a calcined product which generates photons.

### Means for Solving the Problems

The present invention is, for example, the following items [1] to [8].
[1] A calcined product of a composition which comprises (i) alumina, (ii) at least one kind selected from the group consisting of silica and titanium oxide, (iii) at least one kind selected from the group consisting of platinum, a platinum compound, palladium, a palladium compound, iridium, an iridium compound, rhodium, and a rhodium compound, and (iv) silicon nitride,
   wherein the composition contains the silicon nitride (iv) in an amount of 5 to 10 parts by mass, in 100 parts by mass of the composition.
[2] The calcined product according to item [1] above, which further comprises (v) at least one kind selected from the group consisting of silver, a silver compound, gold, and a gold compound.
[3] The calcined product according to item [1] above, which further comprises (vi) celsian.
[4] The calcined product according to item [1] above, which further comprises (vii) steatite.
[5] The calcined product according to item [1] above, which is in the form of particles having an average particle diameter of less than 0.1 µm.
[6] The calcined product according to item [1] above, wherein the component (iii) comprises platinum or a platinum compound.
[7] A fiber comprising the calcined product according to any one of items [1] to [6] above.
[8] A film comprising the calcined product according to any one of items [1] to [6] above.

### Effect of the Invention

The calcined product of the present invention generates photons.

### BRIEF DESCRIPTION OF THE DRAWINGS

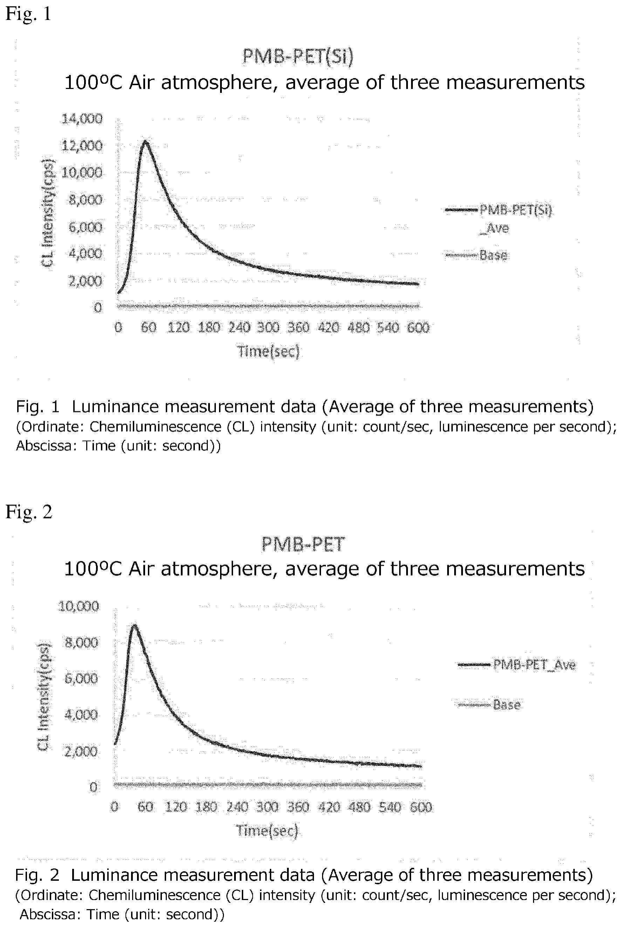

[Fig. 1] A diagram showing the results of measurement of photons generated from the calcined product obtained in Example 1 (average particle diameter: less than 0.1 µm) using Chemiluminescence Analyzer (weak-luminescence spectrometer) CLA-FS4 (manufactured by Tohoku Electronic Industrial Co., Ltd.) and sample chamber CLS-ST4 (manufactured by Tohoku Electronic Industrial Co., Ltd.).
[Fig. 2] A diagram showing the results of measurement of photons generated from the calcined product obtained in Comparative Example 1 (average particle diameter: less than 0.1 µm) using Chemiluminescence Analyzer (weak-luminescence spectrometer) CLA-FS4 (manufactured by Tohoku Electronic Industrial Co., Ltd.) and sample chamber CLS-ST4 (manufactured by Tohoku Electronic Industrial Co., Ltd.).

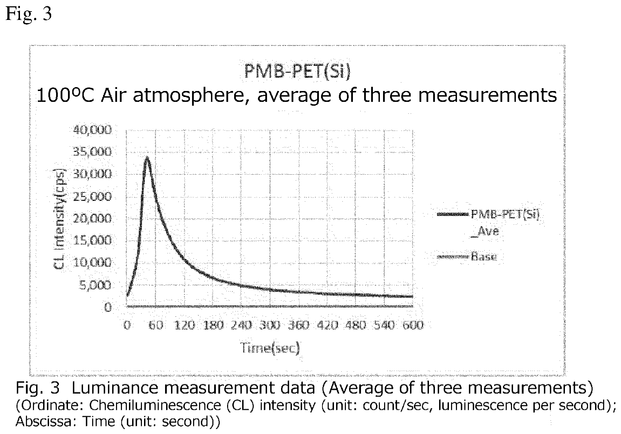

[Fig. 3] A diagram showing the results of measurement of photons generated from the calcined product obtained in Example 1 (average particle diameter: less than 0.1 µm) using Chemiluminescence Analyzer (weak-luminescence spectrometer) CLA-FS5 (manufactured by Tohoku Electronic Industrial Co., Ltd.) and sample chamber CLS-ST5 (manufactured by Tohoku Electronic Industrial Co., Ltd.).

### MODE FOR CARRYING OUT THE INVENTION

The present invention is directed to a calcined product of a composition which comprises (i) alumina, (ii) at least one kind selected from the group consisting of silica and titanium oxide, (iii) at least one kind selected from the group consisting of platinum, a platinum compound, palladium, a palladium compound, iridium, an iridium compound, rhodium, and a rhodium compound, and (iv) silicon nitride, wherein
the composition contains the silicon nitride (iv) in an amount of 5 to 10 parts by mass, in 100 parts by mass of the composition.

It is preferred that the composition of the present invention further comprises (v) at least one kind selected from the group consisting of silver, a silver compound, gold, and a gold compound.

### <Component (i): Alumina>

The composition constituting the calcined product of the present invention comprises alumina.

With respect to the "alumina" (Al₂O₃), high-purity alumina (aluminum oxide) having a purity of 99.9% or more and having excellent sintering properties is preferably used. As the alumina, a commercially available high-purity alumina in a powdery form can be used. Alumina is porous, and therefore is considered to exhibit properties of the component itself and further have such an effect that alumina has supported thereon component (iii) and optional component (vi).

Further, the average particle diameter of component (i) varies depending on the product using the calcined product of the present invention and the form of the calcined product used, but one having a general particle diameter, for example, a particle diameter of 0.1 to several µm can be used. When used in the form of being mixed into a fiber, the average particle diameter of component (i) varies depending on the fiber diameter, and is preferably adjusted to 2 µm or less, more preferably 1.5 µm or less, further preferably less than 1.0 µm, for example, a particle diameter of about 0.3 µm. In the present specification, the average particle diameter is a value determined by a laser diffraction scattering method.

The amount of component (i) contained is preferably 20 to 60 parts by mass, more preferably 25 to 50 parts by mass, in 100 parts by mass of the composition. When the amount of component (i) contained is in the above range, generation of photons can be improved. In the present specification, the wording "100 parts by mass of the composition" means 100 parts by mass of the solid components of the composition, and does not include a liquid material, for example, a suspending medium for component (iii) in a colloidal form.

### <Component (ii): At least one kind selected from the group consisting of silica and titanium oxide>

The composition constituting the calcined product of the present invention comprises at least one kind selected from the group consisting of silica and titanium oxide.

The "silica" (SiO₂) is preferably high-purity silica having a purity of 99.8% or more, and, for example, commercially available anhydrous silica in an ultrafine particle form can be used. The average particle diameter of the silica is similar to that of component (i).

The "titanium oxide" means titanium(IV) oxide (TiO₂). The average particle diameter of the titanium oxide is similar to that of component (i).

Silica and titanium oxide are porous, and therefore are considered to exhibit physical properties of each component itself and further have such an effect that silica or titanium oxide has supported thereon component (iii) and optional component (vi). Titanium oxide is considered to also function as a photocatalyst.

With respect to component (ii), it is preferred that silica and titanium oxide are used in combination.

The amount of component (ii) contained is preferably 30 to 70 parts by mass, more preferably 40 to 65 parts by mass, in 100 parts by mass of the composition. When the amount of component (ii) contained is in the above range, generation of photons can be improved.

### <Component (iii): At least one kind selected from the group consisting of platinum, a platinum compound, palladium, a palladium compound, iridium, an iridium compound, rhodium, and a rhodium compound>

The composition constituting the calcined product of the present invention comprises at least one kind selected from the group consisting of platinum, a platinum compound, palladium, a palladium compound, iridium, an iridium compound, rhodium, and a rhodium compound. Of these, the composition preferably comprises at least one kind selected from the group consisting of at least platinum and a platinum compound.

The at least one kind selected from the group consisting of platinum, a platinum compound, palladium, a palladium compound, iridium, an iridium compound, rhodium, and a rhodium compound is considered to be a main component of the calcined product of the present invention for generating photons.

With respect to the platinum compound, palladium compound, iridium compound, and rhodium compound, examples of forms of the compound include an organic acid salt, an inorganic acid salt, a halide, an oxide, a hydroxide, and an ammonia compound.

Component (iii) is preferably added in the form of a colloid. This is because so-called colloid activation that adsorbs oxygen and hydrogen can be expected. With respect to component (iii), there is preferably used a dispersed colloid of component (iii), which has component (iii) having an average particle diameter of about 0.7 to 4 nm (7 to 40 Å) colloid-dispersed in, for example, a hydrochloric acid solution. As a method for preparing the colloid of component (iii), a common method can be used. With respect to the colloid of component (iii), for example, a commercially available platinum colloidal solution containing, for example, platinum in an amount of 1% by mass can be used.

From the viewpoint of colloid activation, the colloid of component (iii) comprising therein component (iii) preferably in an amount of 0.1 to 5% by mass, more preferably 0.5 to 2% by mass, further preferably 0.8 to 1.2% by mass is used.

Component (iii) is preferably contained in an amount of 0.0015 to 0.0500 parts by mass, more preferably 0.003 to 0.030 parts by mass, in terms of the amount of a metal, in 100 parts by mass of the composition of the present invention. When the amount of component (iii) contained is in the above range, a calcined product of the resultant composition can generate photons enough to exhibit, for example, effects for promotion of health.

### <Component (iv): Silicon nitride>

The composition constituting the calcined product of the present invention comprises silicon nitride.

Silicon nitride as an optional component is a ceramic having a formulation: Si₃N₄. The ceramic is obtained by sintering preferably at 1,780°C. Silicon nitride is considered to have a role in making hydrogen active to control the direction of movement of hydrogen ions to be in a specific direction. In addition, silicon nitride is considered to exhibit physical properties of the component itself and further have such an effect that silicon nitride has supported thereon component (iii) and optional component (v).

Component (iv) is contained in an amount of 5 to 10 parts by mass, preferably 5 to 7 parts by mass, in 100 parts by mass of the composition of the present invention. When the amount of component (iv) contained is in the above range, generation of photons can be improved.

### <Component (v): At least one kind selected from the group consisting of silver, a silver compound, gold, and a gold compound>

It is preferred that the composition constituting the calcined product of the present invention comprises at least one kind selected from the group consisting of silver, a silver compound, gold, and a gold compound.

Optional component (v) is preferably used in the form of a powder, and a commercially available powder of silver, gold, or a compound thereof can be used. Optional component (v) more preferably comprises at least one kind selected from the group consisting of silver and a silver compound.

Further, the average particle diameter of component (v) varies depending on the product using the calcined product of the present invention and the form of the calcined product used, but is preferably adjusted to 2 µm or less, more preferably 1.5 µm or less, further preferably 1.0 µm or less, for example, a particle diameter of 0.7 µm.

Component (v) is preferably contained in an amount of 0 to 30.0 parts by mass, more preferably 1.5 to 30.0 parts by mass, further preferably 1.5 to 15.0 parts by mass, especially preferably 2.1 to 6.0 parts by mass, in terms of the amount of gold or silver, in 100 parts by mass of the composition of the present invention. When the amount of component (v) contained is in the above range, generation of photons can be improved.

The composition constituting the calcined product of the present invention may further comprise component (vi) celsian and/or component (vii) steatite.

### <Component (vi): Celsian>

Celsian as an optional component is a ceramic having a structure: BaO·Al₂O₃·2SiO₂. As an example of the method for producing celsian, there can be mentioned a known method for producing celsian, and, for example, there can be mentioned calcination conducted using BaCO₃, α-Al₂O₃, and SiO₂ as raw materials.

Component (vi) is preferably contained in an amount of 0.1 to 5.0 parts by mass, more preferably 1.0 to 2.5 parts by mass, in 100 parts by mass of the composition of the present invention.

### <Component (vii): Steatite>

Steatite as an optional component is a ceramic having a structure: MgO·SiO₂, and is preferably protoenstatite. As an example of the method for producing steatite, there can be mentioned a known method for producing steatite, and, for example, there can be mentioned calcination conducted using talc as a raw material.

Component (vii) is preferably contained in an amount of 0.1 to 5.0 parts by mass, more preferably 1.0 to 2.5 parts by mass, in 100 parts by mass of the composition of the present invention.

### <Other components>

The composition constituting the calcined product of the present invention may contain an additive, such as a binder or a stabilizer, in addition to the above-mentioned components.

### <Method for producing the calcined product>

The calcined product of the present invention can be produced by a method which comprises the steps of:
(step 1) mixing components (i), (ii), and (iv) to obtain a first mixture;
(step 2) adding component (iii) and an optional component to the first mixture obtained in step 1 above to obtain a second mixture; and
(step 3) heating the second mixture obtained in step 2 above at 800 to 1,000°C to obtain a calcined product.

The mixing step (step 1) is first conducted. Specifically, components (i), (ii), and (iv) are mixed to obtain a first mixture.

Then, the mixing step (step 2) is conducted. Specifically, component (iii) and an optional component are mixed with the first mixture obtained in step 1 to obtain a second mixture. In this step, component (iii) and optional component (v) may be heated and brought into contact with the first mixture, and it is preferred that the temperature or pressure in this instance is controlled to cause the pores and/or surface of components (i), (ii), and (iv) to have supported thereon component (iii) and optional component (v).

Finally, the calcination step (step 3) is conducted. Specifically, the second mixture obtained in step 2 above is heated at 800 to 1,000°C for 15 to 20 minutes to obtain a calcined product.

Further, the calcined product of the present invention can be produced in the form of a finely divided particle powder. The calcined product of the present invention is preferably in the form of particles having an average particle diameter of less than 0.1 µm. It is considered that when the average particle diameter of the calcined product is in the above-mentioned range, the amount of photons generated is increased. The average particle diameter is a value determined by a laser diffraction scattering method.

As examples of methods for obtaining the calcined product having the above-mentioned average particle diameter, there can be mentioned a method of controlling the particle diameter of a raw material for the calcined product, and a method of subjecting the calcined product to wet milling in the presence of alumina balls using a bead mill.

### <Properties>

As shown in Figs. 1 and 3, the calcined product of the present invention emits a light. That is, the calcined product generates photons.

### <Use>

The calcined product of the present invention is used in the application of promotion of health.

More specifically, there can be mentioned applications of promotion of health, such as an improvement of blood circulation, suppression of a lipoperoxide, suppression of formation of lactic acid after exercise, and stabilization of immunity. Further specifically, the calcined product is expected to assist in curing, for example, menstrual pain, being sensitive to the cold, stiffness of the shoulders, rheumatism, arteriosclerosis, hypercholesterolemia, diabetes, fatigue recovery, prevention of influenza, or microthrombus.

The calcined product of the present invention generates photons as mentioned above, and therefore it is considered that when using the calcined product of the present invention, NO is generated in a human body, and that the generated NO relaxes blood vessels to affect blood circulation and others, and thus the calcined product can be advantageously used in the above-mentioned applications of promotion of health.

### <Product>

With respect to the calcined product of the present invention, for example, a fiber or a film containing therein the calcined product can be obtained.

When obtaining a chemical fiber or film containing therein the calcined product, a masterbatch containing the calcined product of the present invention can be used. As examples of methods for producing a masterbatch, there can be mentioned a method in which the calcined product of the present invention, which is in the form of a powder, is incorporated into a synthetic polymer material, and a method in which the calcined product, which is in the form of a dispersion, is mixed with a synthetic polymer material. In this instance, the amount of the calcined product of the present invention in the masterbatch can be 0.1 to 25% by mass.

### 1. Fiber

The calcined product of the present invention can be in the form of a product having the calcined product contained in a fiber.

Examples of fibers include natural fibers and chemical fibers, and examples of natural fibers include cotton, wool, silk, and linen, and examples of chemical fibers include a cellulose fiber, a polyamide fiber, such as nylon, a polyvinyl alcohol fiber, such as vinylon, a polyvinylidene chloride fiber, a polyvinyl chloride fiber, a polyester fiber, an acrylic fiber, a polyolefin fiber, and a polyurethane fiber. These fibers may be used individually or in combination. For example, a mixed fiber having mixed a natural fiber and a chemical fiber containing the calcined product of the present invention can be used.

For obtaining a fiber containing therein the calcined product of the present invention, the calcined product of the present invention can be incorporated into a fiber or applied to the surface of a fiber. As an example of a method for incorporating the calcined product of the present invention into a fiber, there can be mentioned a method in which the calcined product of the present invention is mixed in an amount of, for example, 0.1 to 25% by mass, preferably 0.1 to 3% by mass, more preferably 0.3 to 1.5% by mass, into a fiber, and the fiber is melted to form a mixture, and the mixture is spun using a common spinning method, for example, melt spinning, dry spinning, or wet spinning. It is preferred that a masterbatch containing the calcined product of the present invention is produced and then spun. As a method for applying the calcined product of the present invention to the surface of a fiber, a spraying, dipping, or coating method can be employed.

The calcined product of the present invention and, for example, an enzyme or a humectant, such as hyaluronic acid, can be incorporated into a fiber.

The fiber containing therein the calcined product of the present invention is advantageously used in, for example, bedding articles and clothing.

### 2. Film

The calcined product of the present invention can be in the form of a product having the calcined product contained in a film.

Examples of resins for the film include thermoplastic resins, such as polyolefin, polyvinyl chloride, polyvinylidene chloride, polyethylene terephthalate, and polyurethane.

As an example of the method for obtaining a film containing therein the calcined product of the present invention, there can be mentioned a method in which the calcined product of the present invention in a powdery form is added to and mixed with the above-mentioned resin in a molten state, and then formed by a known method. A masterbatch containing the calcined product of the present invention can be mixed with the above-mentioned thermoplastic resin, followed by forming.

The film containing the calcined product of the present invention is advantageously used in a surgical tape.

In the production of the above-mentioned fiber or film, if necessary, various types of additives, for example, magnesium oxide, mica, calcium carbonate, a catalyst, such as zeolite, a plasticizer, an ultraviolet light absorber, a filler, a colorant, a color protection agent, a flame retardant, a bleed-out preventive agent, a stabilizer, a heat-resistant agent, or a fluorescent brightener can be added.

### Examples

Hereinbelow, the present invention will be described in more detail with reference to the following Examples and Comparative Examples, which should not be construed as limiting the scope of the present invention.

### [Example 1] Production of a calcined product (1)

With respect to commercially available alumina, silica, titanium oxide (titania), and silicon nitride, the particle size of each one was adjusted to a particle size of less than 1 µm. Then, 30 parts by mass of the alumina, 30 parts by mass of the silica, 30 parts by mass of the titanium oxide (titania), and 5 parts by mass of the silicon nitride were mixed to obtain a first mixture.

To the first mixture were added 2.5 parts by mass (0.025 parts by mass, in terms of the amount of platinum) of a platinum colloidal solution containing platinum in an amount of 1% (average particle diameter: 40 angstroms) and 2.5 parts by mass of a silver powder having a particle diameter of 0.2 to 1.0 µm and an average particle diameter of 0.7 µm, obtaining a second mixture. The second mixture was heated at 800°C for 20 minutes to obtain a calcined product.

The obtained calcined product was pulverized by wet milling in the presence of alumina balls using an apparatus called a bead mill. The resultant pulverized calcined product had an average particle diameter of less than 0.1 µm, as determined by a laser diffraction scattering method.

With respect to the calcined product having an average particle diameter of less than 0.1 µm, a chemiluminescence (CL) intensity was measured. The results are shown in Fig. 1.

### [Comparative Example 1] Production of a calcined product (2)

A calcined product having an average particle diameter of less than 0.1 µm was obtained in substantially the same manner as in Example 1 except that silicon nitride was not used and 31.67 parts by mass of the alumina, 31.67 parts by mass of the silica, and 31.67 parts by mass of the titanium oxide (titania) were mixed to obtain a first mixture.

With respect to the calcined product having an average particle diameter of less than 0.1 µm and containing no silicon nitride, a chemiluminescence (CL) intensity was measured. The results are shown in Fig. 2.

### [Example 2] Production of a calcined product (3)

With respect to commercially available alumina, silica, titanium oxide (titania), and silicon nitride, the particle size of each one was adjusted to a particle size of 1 µm or less. Then, 30 parts by mass of the alumina, 30 parts by mass of the silica, 30 parts by mass of the titanium oxide (titania), and 7 parts by mass of the silicon nitride were mixed to obtain a first mixture.

To the first mixture were added 2.5 parts by mass (0.025 parts by mass, in terms of the amount of platinum) of a platinum colloidal solution containing platinum in an amount of 1% (average particle diameter: 40 angstroms), 2.5 parts by mass of a silver powder having a particle diameter of 0.2 to 1.0 µm and an average particle diameter of 0.7 µm, 1.5 parts by weight of pulverized celsian (BaO·Al₂O₃·2SiO₂), and 1.5 parts by weight of pulverized protoenstatite, obtaining a second mixture.

The second mixture was heated at 800°C for 20 minutes to obtain a calcined product.

The obtained calcined product was pulverized by wet milling in the presence of alumina balls using an apparatus called a bead mill. The resultant pulverized calcined product had an average particle diameter of less than 0.1 µm, as determined by a laser diffraction scattering method.

### [Application Example 1]

6 Parts by mass of the calcined product in Example 1 was mixed with 100 parts by mass of polyester to prepare a masterbatch. The masterbatch was mixed with polyester in an amount of 10% by mass, and subjected to melt spinning, producing a 75 denier yarn and a 30 denier yarn.

### [Application Example 2]

Using the fiber obtained in Application Example 1, the following cloth was produced.
Fiber mixing ratio: the fiber in Application Example 1: 30%; cotton: 70%

### [Application Example 3]

Using the fiber obtained in Application Example 1, the following cloth was produced.
Fiber mixing ratio: the fiber in Application Example 1: 100%

The values in the Examples were individually measured by the methods described below.
[Measurement of chemiluminescence (CL) intensity for Figs. 1 and 2] 6 Parts by mass of the calcined product in Example 1 was mixed with 100 parts by mass of polyester to prepare a masterbatch, and the masterbatch was used in the measurement.
Measuring apparatus: Chemiluminescence Analyzer (weak-luminescence spectrometer) CLA-FS4 (manufactured by Tohoku Electronic Industrial Co., Ltd.; luminescence detection region: 300 to 850 nm; central wavelength: 420 nm) and sample chamber CLS-ST4 (manufactured by Tohoku Electronic Industrial Co., Ltd.) were used. Conditions for measurement: 2 g of the masterbatch was placed in a ϕ50 stainless steel Petri dish, and a chemiluminescence (CL) intensity was measured in air at 100°C for 10 minutes.

The measurement was conducted three times, and an average of the measured values is shown in Fig. 1.

With respect to the calcined product in Comparative Example 1, a chemiluminescence (CL) intensity was similarly measured. The results are shown in Fig. 2.

[Measurement of chemiluminescence (CL) intensity for Fig. 3] 6 Parts by mass of the calcined product in Example 1 was mixed with 100 parts by mass of polyester to prepare a masterbatch, and the masterbatch was used in the measurement.
Measuring apparatus: Chemiluminescence Analyzer (weak-luminescence spectrometer) CLA-FS5 (manufactured by Tohoku Electronic Industrial Co., Ltd.; luminescence detection region: 300 to 650 nm; central wavelength: 420 nm) and sample chamber CLS-ST5 (manufactured by Tohoku Electronic Industrial Co., Ltd.) were used. Conditions for measurement: 2 g of the masterbatch was placed in a ϕ50 stainless steel Petri dish, and a chemiluminescence (CL) intensity was measured in air at 100°C for 10 minutes.

The measurement was conducted three times, and an average of the measured values is shown in Fig. 3.

### [Measurement of an average particle diameter]

An average particle diameter was determined by a laser diffraction scattering method.

From a comparison between Fig. 1 and Fig. 2, it is apparent that the peak value of the chemiluminescence (CL) intensity of photons of the calcined product of the present invention shown in Fig. 1 is 1.3 times higher than that of the calcined product of Fig. 2 containing no silicon nitride.

A comparison between Fig. 1 and Fig. 3 shows that when using Chemiluminescence Analyzer (weak-luminescence spectrometer) CLA-FS5 as a measuring apparatus, instead of Chemiluminescence Analyzer (weak-luminescence spectrometer) CLA-FS4, the peak value of the chemiluminescence (CL) intensity of photons is increased two or more times. Chemiluminescence Analyzer CLA-FS5 is narrowed in the luminescence detection region, as compared to Chemiluminescence Analyzer CLA-FS4, and thus is further improved in the ability of luminescence detection with respect to the central wavelength portion, and has an improved sensitivity.

## Claims

1. A calcined product of a composition which comprises (i) alumina, (ii) at least one kind selected from the group consisting of silica and titanium oxide, (iii) at least one kind selected from the group consisting of platinum, a platinum compound, palladium, a palladium compound, iridium, an iridium compound, rhodium, and a rhodium compound, and (iv) silicon nitride, wherein
the composition contains the silicon nitride (iv) in an amount of 5 to 10 parts by mass, in 100 parts by mass of the composition.

2. The calcined product according to claim 1, which further comprises (v) at least one kind selected from the group consisting of silver, a silver compound, gold, and a gold compound.

3. The calcined product according to claim 1, which further comprises (vi) celsian.

4. The calcined product according to claim 1, which further comprises (vii) steatite.

5. The calcined product according to claim 1, which is in the form of particles having an average particle diameter of less than 0.1 µm.

6. The calcined product according to claim 1, wherein the component (iii) comprises platinum or a platinum compound.

7. A fiber comprising the calcined product according to any one of claims 1 to 6.

8. A film comprising the calcined product according to any one of claims 1 to 6.
